# EUROPÄISCHE PATENTSCHRIFT

(11) **EP 2 151 255 B1**
(45) Veröffentlichungstag und Bekanntmachung des Hinweises auf die Patenterteilung: **09.08.2017**
(21) Anmeldenummer: 09163981.5
(22) Anmeldetag: 29.06.2009
(51) Int. Cl.: A61L 31/02, A61L 31/10, A61L 31/14, A61L 31/16

(54) **Biokorrodierbares Implantat mit einer Beschichtung enthaltend ein Hydrogel**
Biocorrodible implant with a coating comprising a hydrogel
Implant biocorrodable recouvert d'un revêtement hydrogel

(30) Priorität: 28.07.2008 DE 102008040787
(43) Veröffentlichungstag der Anmeldung: 10.02.2010
(73) Patentinhaber: Biotronik VI Patent AG, 6341 Baar (CH)
(72) Erfinder: Borck, Alexander, 91086, Aurachtal (DE); Adden, Nina, 90427, Nürnberg (DE)
(74) Vertreter: Randoll, Sören

(56) Entgegenhaltungen:
- EP-A2- 1 795 215
- WO-A1-02/05864
- US-A1- 2006 165 751
- US-A1- 2008 058 923

## Beschreibung

### Technisches Gebiet

Die Erfindung betrifft ein biokorrodierbares Implantat mit einer Beschichtung enthaltend ein Hydrogel.

### Technologischer Hintergrund und Stand der Technik

Implantate haben in vielfältiger Ausführungsform Anwendung in der modernen Medizintechnik gefunden. Sie dienen beispielsweise der Unterstützung von Gefäßen, Hohlorganen und Gangsystemen (endovaskuläre Implantate), zur Befestigung und temporären Fixierung von Gewebeimplantaten und Gewebstransplantationen, aber auch zu orthopädischen Zwecken, z.B. als Nagel, Platte oder Schraube.

So hat sich zum Beispiel die Implantation von Stents als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Stents besitzen eine Umfangswandung von ausreichender Tragkraft, um das verengte Gefäß im gewünschten Maße offen zu halten, und einen rohrförmigen Grundkörper durch den der Blutfluss ungehindert weiterläuft. Die Umfangswandung wird in der Regel von einer gitterartigen Tragstruktur gebildet, die es erlaubt, den Stent in einem komprimierten Zustand mit kleinem Außendurchmesser bis zur zu behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist. Der Vorgang der Positionierung und Expansion der Stents während der Prozedur und die abschließende Lage des Stents im Gewebe nach Beendigung der Prozedur muss durch den Kardiologen überwacht werden. Dies kann durch bildgebende Verfahren, wie z.B. durch Röntgenuntersuchungen erfolgen.

Das Implantat oder der Stent besitzt einen Grundkörper aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßer Verwendung mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und degradierbare/resorbierbare Werkstoffe unterteilt werden.

Eine biologische Reaktion auf polymere, keramische oder metallische Implantatwerkstoffe hängt von der Konzentration, Einwirkdauer und Art der Zuführung ab. Häufig führt die Gegenwart eines Implantatwerkstoffs zu Entzündungsreaktionen, deren Auslöser mechanische Reize, chemische Stoffe aber auch Stoffwechselprodukte sein können. Der Entzündungsvorgang ist in der Regel begleitet von der Einwanderung neutrophiler Granulozyten und Monozyten durch die Gefäßwände, der Einwanderung von Lymphozyten-Effektorzellen unter Bildung spezifischer Antikörper gegen den Entzündungsreiz, der Aktivierung des Komplementsystems unter Freisetzung von Komplementfaktoren, die als Mediatoren wirken, und letztendlich der Aktivierung der Blutgerinnung. Eine immunologische Reaktion ist meist eng mit der Entzündungsreaktion verbunden und kann zur Sensibilisierung und Allergiebildung führen. Bekannte metallische Allergene umfassen beispielsweise Nickel, Chrom und Kobalt, die auch in vielen chirurgischen Implantaten als Legierungskomponenten Verwendung finden. Ein wesentliches Problem der Stentimplantation in Blutgefäße ist die In-Stent Restenose aufgrund eines überschießenden neointimalen Wachstums, das durch eine starke Proliferation der arteriellen glatten Muskelzellen und eine chronische Entzündungsreaktion hervorgerufen wird.

Ein aussichtsreicher Ansatz zur Lösung des Problems liegt in der Verwendung biokorrodierbarer Metalle und deren Legierungen als Implantatwerkstoff, denn zumeist ist eine dauerhafte Stützfunktion durch den Stent nicht erforderlich; das zunächst geschädigte Körpergewebe regeneriert. So wird beispielsweise in DE 197 31 021 A1 vorgeschlagen, medizinische Implantate aus einem metallischen Werkstoff zu formen, dessen Hauptbestandteil Eisen, Zink oder Aluminium sind bzw. ein Element aus der Gruppe der Alkalimetalle oder Erdalkalimetalle. Als besonders geeignet werden Legierungen auf Basis von Magnesium, Eisen und Zink beschrieben. Nebenbestandteile der Legierungen können Mangan, Kobalt, Nickel, Chrom, Kupfer, Cadmium, Blei, Zinn, Thorium, Zirkonium, Silber, Gold, Palladium, Platin, Silizium, Calcium, Lithium, Aluminium, Zink und Eisen sein. Weiterhin ist aus der DE 102 53 634 A1 der Einsatz einer biokorrodierbaren Magnesiumlegierung mit einem Anteil von Magnesium >90 %, Yttrium 3,7 - 5,5 %, Seltenerdmetallen 1,5 - 4,4 % und Rest <1 % bekannt, die sich insbesondere zur Herstellung einer Endoprothese, zum Beispiel in Form eines selbstexpandierenden oder ballonexpandierbaren Stents, eignet. Der Einsatz von biokorrodierbaren metallischen Werkstoffen in Implantaten dürfte zu einer deutlichen Minderung von Abstoßungs- oder Entzündungsreaktionen führen. Solche biokorrodierbaren Implantate und Stents weisen häufig auch eine Beschichtung oder Kavitätenfüllung mit einem geeigneten Polymer auf.

Ein Problem beim Einsatz dieser biokorrodierbaren Implantate, die ganz oder in Teilen aus einem metallischen Werkstoff bestehen, liegt darin, dass die Abbauprodukte, die bei der Korrosion des Implantats entstehen und freigesetzt werden, oft einen merklichen Einfluss auf den lokalen pH-Wert haben und sowohl zu ungewünschten Gewebsreaktionen führen können, als auch einen unerwünschten Einfluss auf die weitere Korrosionsrate des Implantats ausüben können. Insbesondere beim Abbau von Mg-haltigen biokorrodierbaren Implantatwerkstoffen kann es zu einem Anstieg des pH-Wertes in der unmittelbaren Umgebung kommen. Dieser lokale pH-Wertanstieg führt dabei zu einem Ungleichgewicht der Ladungsverteilung in den das Gefäß umgebenden Muskelzellen, was dazu führen kann, dass es zu einer lokalen Erhöhung des Muskeltonus im Bereich des Implantats kommt. Dieser erhöhte Druck auf das Implantat kann zum vorzeitigen Verlust der Implantatintegrität führen.

Dokument EP 1 795 215 A2 offenbart ein Implantat (Stent) mit einem Grundkörper, der ganz oder in Teilen aus einem biokorrodierbaren, metallischen Werkstoff besteht (Magnesium), wobei der Werkstoff so beschaffen ist, dass er in wässriger Umgebung zu einem alkalischen Produkt zersetzt wird und wobei der Grundkörper eine Beschichtung aufweist, die aus einem säureabgebenden Polymer besteht oder dieses enthält. Daneben ist es wünschenswert die Geschwindigkeit der Korrosion des eingesetzten biokorrodierbaren Implantats beeinflussen und vorab gezielt festlegen zu können.

Aufgabe der vorliegenden Erfindung war es einen oder mehrere der geschilderten Nachteile des Standes der Technik zu mindern oder zu überwinden.

### Erfindungsgemäße Lösung

Die Aufgabe wird durch Bereitstellung eines Implantats gelöst, mit einem Grundkörper, der ganz oder in Teilen aus einem biokorrodierbaren, metallischen Werkstoff besteht, wobei der Werkstoff so beschaffen ist, dass er in wässriger Umgebung zu einem alkalischen Produkt zersetzt wird und wobei der Grundkörper eine Beschichtung aufweist, die aus einem Hydrogel besteht oder dieses enthält, dadurch gekennzeichnet, dass das Hydrogel bei erhöhtem pH-Wert ein verringertes Quellvermögen besitzt.

Ein Vorteil der erfindungsgemäßen Lösung liegt darin, dass bei Vorliegen eines lokal erhöhten pH-Wertes, das Quellvermögen des Hydrogels in der Implantatbeschichtung verringert ist. Das Hydrogel nimmt bei erhöhtem pH-Wert weniger Wasser auf. Dies führt zu einer Volumenverringerung des Hydrogels und damit zu einer Volumenabnahme der das Hydrogel enthaltenden Beschichtung des Implantats. In der Folge legt sich das Hydrogel nun enger um das Implantat, verringert damit die Menge an Oxidationspartnern pro Zeit, die für die Korrosion der Implantats zur Verfügung stehen und verlangsamt so die Korrosionsrate des biokorrodierbaren Implantats. Damit kann sich der lokale pH-Wert wieder normalisieren, es kommt nicht zu länger anhaltenden Phasen, in denen der lokale pH-Wert erhöht ist. Damit ist die Gefahr der Ausbildung einer Alkalose deutlich verringert. Bei Einsatz des erfindungsgemäßen Implantats nimmt somit die Inzidenz an klinischen Alkalosen ab. Zur begleitenden Behandlung bzw. Prophylaxe ist eine medikamentöse Behandlung nicht mehr geboten, bzw. es können geringere Dosen an Wirkstoffen eingesetzt werden. Der Patient wird weniger belastet und die Behandlungskosten sind reduziert.

Implantate im Sinne der Erfindung sind über ein chirurgisches Verfahren in den Körper eingebrachte Vorrichtungen und umfassen Befestigungselemente für Knochen, beispielsweise Schrauben, Platten oder Nägel, chirurgisches Nahtmaterial, Darmklammern, Gefäßclips, Prothesen im Bereich des Hart- und Weichgewebes und Ankerelemente für Elektroden, insbesondere von Schrittmachern oder Defibrillatoren.

Vorzugsweise ist das Implantat ein Stent. Stents herkömmlicher Bauart weisen eine filigrane Stützstruktur aus metallischen Streben auf, die zur Einbringung in den Körper zunächst in einem nicht-expandierten Zustand vorliegt und die am Ort der Applikation dann in einen expandierten Zustand aufgeweitet wird. Der Stent kann vor oder nach dem Crimpen auf einen Ballon beschichtet werden.

Gemäß einer ersten Variante weist der Grundkörper des Implantats demnach eine Beschichtung auf, die ein erfindungsgemäßes Hydrogel enthält oder aus diesem besteht. Eine Beschichtung im Sinne der Erfindung ist eine zumindest abschnittsweise Auftragung der Komponenten der Beschichtung auf den Grundkörper des Implantats. Vorzugsweise wird die gesamte Oberfläche des Grundkörpers des Implantats von der Beschichtung bedeckt. Eine Schichtdicke liegt vorzugsweise im Bereich von 1 nm bis 100 µm, besonders bevorzugt 300 nm bis 30 µm. Der Gewichtsanteil des erfindungsgemäßen Hydrogels an den die Beschichtung bildenden Komponenten der Beschichtung beträgt vorzugsweise mindestens 40%, besonders bevorzugt mindestens 70%. Die Beschichtung kann direkt auf die Implantatoberfläche aufgetragen werden. Die Verarbeitung kann nach Standardverfahren für die Beschichtung erfolgen. Es können einschichtige, aber auch mehrschichtige Systeme (zum Beispiel so genannte base coat-, drug coat- oder top coat-Schichten) erstellt werden. Die Beschichtung kann unmittelbar auf dem Grundkörper des Implantats aufgebracht sein oder es sind weitere Schichten dazwischen vorgesehen, die beispielsweise der Haftvermittlung dienen.

Als biokorrodierbar im Sinne der Erfindung werden Legierungen und Elemente bezeichnet, bei denen in physiologischer Umgebung ein Abbau/Umbau stattfindet, so dass der aus dem Werkstoff bestehende Teil des Implantates ganz oder zumindest überwiegend nicht mehr vorhanden ist. Biokorrodierbare metallische Werkstoffe im Sinne der Erfindung umfassen Metalle und Legierungen ausgewählt aus der Gruppe umfassend Eisen, Wolfram, Zink, Molybdän und Magnesium und insbesondere solche biokorrodierbare metallische Werkstoffe, die in wässriger Lösung zu einem alkalischen Produkt korrodieren.

Vorzugsweise besteht der metallische Grundkörper aus Magnesium, einer biokorrodierbaren Magnesiumlegierung, Reineisen, einer biokorrodierbaren Eisenlegierung, einer biokorrodierbaren Wolframlegierung, einer biokorrodierbaren Zinklegierung oder einer biokorrodierbaren Molybdänlegierung. Insbesondere ist der biokorrodierbare metallische Werkstoff eine Magnesiumlegierung.

Unter einer biokorrodierbare Magnesiumlegierung wird ein metallisches Gefüge verstanden, dessen Hauptkomponente Magnesium ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Vorzugsweise enthält die biokorrodierbare Magnesiumlegierung Yttrium und weitere Seltenerdmetalle, da sich eine derartige Legierung aufgrund ihrer physikochemischen Eigenschaften und hohen Biokompatibilität, insbesondere auch seiner Abbauprodukte, auszeichnet. Besonders bevorzugt wird eine Magnesiumlegierung der Zusammensetzung Seltenerdmetalle 5,2 - 9,9 Gew.%, davon Yttrium 3,7 - 5,5 Gew.%, und Rest < 1 Gew.%, wobei Magnesium den auf 100 Gew.% fehlenden Anteil an der Legierung einnimmt, eingesetzt. Diese Magnesiumlegierung bestätigte bereits experimentell und in ersten klinischen Versuchen ihre besondere Eignung, d. h. zeigt eine hohe Biokompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71) verstanden.

Die Magnesiumlegierung ist so in ihrer Zusammensetzung zu wählen, dass sie biokorrodierbar ist. Als Prüfmedium zur Testung des Korrosionsverhaltens von Legierungen dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCl 0,4 g/l, MgSO₄ 0,1 g/l, NaHCO₃ 2,2 g/l, Na₂HPO₄ 0,126 g/l, NaH₂PO₄ 0,026 g/l). Eine Probe des zu untersuchenden Werkstoffs wird dazu in einem verschlossenen Probenbehälter mit einer definierten Menge des Prüfmediums bei 37°C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Erfindungsgemäß besitzt das Hydrogel der Beschichtung des Implantats bei erhöhtem pH-Wert ein verringertes Quellvermögen.

Ein erhöhter pH-Wert im Sinne der Erfindung liegt dann vor, wenn der lokale pH-Wert, gegenüber dem physiologischen pH-Wert in den basischen pH-Wertbereich verändert ist. Insbesondere liegt dann ein erhöhter pH-Wert im Sinne der Erfindung vor, wenn der pH-Wert in der lokalen Umgebung größer als 8 ist.

Unter dem Begriff "Quellvermögen" im Sinne der Erfindung wird die Eigenschaft des Hydrogels verstanden, eine bestimmte Wassermenge pro mmol Hydrogel-Polymer aufzunehmen. Ein verringertes Quellvermögen führt zu einer Verringerung des Volumens des Hydrogels und damit der dieses Hydrogel enthaltenden Beschichtung des Implantats. Geeignete Methoden und Messverfahren zur Bestimmung des Quellvermögens sind dem Fachmann bekannt, insbesondere eignen sich solche Messverfahren, wie sie sich im Bereich der Galenik bereits vielfach bewährt haben.

In einer bevorzugten Ausführungsform ist das Quellvermögen des Hydrogel bei einem pH-Wert größer als 8 verringert.

In besonders bevorzugten Ausführungsformen ist das Quellvermögen des Hydrogel bei einem pH-Wert größer als 8 derart vermindert, dass das Hydrogel mindestens 30% weniger Wasser pro mmol Hydrogel-Polymer aufnehmen kann als bei physiologischem pH-Wert.

In einer Ausführungsform der Erfindung umfasst die Beschichtung des erfindungsgemäßen Implantats ein Hydrogel. Ein Hydrogel ist ein wasserenthaltendes, aber wasserunlösliches Polymer, dessen Moleküle chemisch, z.B. durch kovalente oder ionische Bindungen oder physikalisch, z.B. durch Verschlaufen der Polymerketten, zu einem dreidimensionalen Netzwerk verknüpft sind. Erfindungsgemäße Hydrogele sind in der Lage bei Änderung des pH-Wertes ihr Volumen zu verändern, in dem sie bei erhöhtem pH-Wert ein verringertes Quellvermögen aufweisen und damit weniger Wasser pro mmol Hydrogel-Polymer aufnehmen können. Diese Hydrogele können beispielsweise hergestellt werden durch Umsetzung ethylenisch ungesättigter Monomere und Polymere die ionisierbare Gruppen tragen mit Vernetzern und Polymerisationskatalysatoren. Alternativ dazu können geeignete Hydrogele hergestellt werden durch Kondensationsreaktionen mit difunktionellen und mehrfunktionellen Monomeren. Dem Fachmann sind geeignete Monomere und Polymere sowie Verfahren zu deren Herstellung bekannt. Ebenso sind dem Fachmann Methoden und Verfahren zur Herstellung von geeigneten Hydrogelen mittels solcher Mono- oder/und Polymere bekannt.

Das Hydrogel der Beschichtung des erfindungsgemäßen Implantats besitzt pH-Wert abhängige Quellungseigenschaften. Bevorzugt weist das Hydrogel mindestens eine funktionelle Gruppe auf, die mit steigendem pH-Wert einen Übergang von einem ionischen hin zu einem neutralen Ladungszustand zeigt. Dabei liegt die mindestens eine funktionelle Gruppe des Hydrogel bei physiologischem pH-Wert in ionischem Ladungszustand und bei, im Sinne der Erfindung, erhöhtem pH-Wert in neutralem Ladungszustand vor. Das Hydrogel des erfindungsgemäßen Implantats kann mehrere gleiche oder verschiedene funktionelle Gruppen aufweisen, die bei physiologischem pH-Wert nicht alle im selben Ladungszustand vorliegen müssen.

In einer bevorzugten Ausführungsform wird die mindestens eine funktionelle Gruppe ausgewählt aus der Gruppe umfassend eine Aminfunktion oder eine Amidfunktion.

Bevorzugte Hydrogele enthalten ein Polymer auf Basis von Acrylamid, Methacrylamid, Dimethylaminoethylmethacrylat oder einem Derivat von Acrylamid, Methacrylamid, oder Dimethylaminoethylmethacrylat.

In einem weiteren Aspekt kann die Beschichtung des erfindungsgemäßen Implantats mindestens einen Wirkstoff enthalten. Als Wirkstoff kann jeder bekannte Wirkstoff eingesetzt werden. Bevorzugt kommen solche Wirkstoffe zum Einsatz, die geeignet sind zur Behandlung oder Prophylaxe einer Alkalose. Solche Wirkstoffe sind ausgewählt aus der Gruppe enthaltend Vasodilatoren, Entzündungshemmer und Wirkstoffe zur lokalen pH-Wertregulation. Besonders bevorzugte Wirkstoffe sind ausgewählt aus der Gruppe enthaltend NO-freisetzende Substanzen und Bosentan®, Dipyridamol, dODN oder allgemein Endothelin-Rezeptor-Antagonisten, Kalziumkanalblocker wie Amlodipin, Nifidipin oder Verapamil.

Das erfindungsgemäße Implantat kann eine weitere, äußere Beschichtung aufweisen. Eine solche weitere äußere Schicht kann die Beschichtung umfassend das Hydrogel ganz oder in Teilen bedecken. Diese äußere Beschichtung besteht bevorzugt aus einem degradierenden Polymer oder enthält dieses, insbesondere ein Polymer aus der Klasse der PLGA (poly(lactic-co-glycolic acid)) oder der PLGA-PEG Blockcopolymere. Ggf. kann in diese weitere, äußere Schicht ein Wirkstoff eingebettet sein, der frei eluieren kann oder bei Degradation der äußeren Beschichtung freigesetzt wird. Eine solche weitere, äußere Beschichtung kann dazu verwendet werden in einem Mehrschichtensystem die durch Änderung des pH-Wertes vermittelte Verringerung der Quellungseigenschaften des Hydrogel und die damit verbundene Volumenreduktion der Beschichtungsschicht zu verzögern. Zunächst wird die weitere äußere Beschichtung degradiert, erst dann ist die innere Beschichtung zugänglich, die dann bei Vorliegen eines erhöhten pH-Wertes eine Volumenverringerung des Hydrogels veranlasst.

Nachfolgend wird die Erfindung anhand von Ausführungsbeispielen näher erläutert.

### Ausführungsbeispiel 1 - Poly(N-isopropylacrylamid-co-allylamine)

3,8g (33,6mmol) N-isopropylacrylamid (NIPAM) und 0,2g (3,4mmol) Allylamin (10% des NIPAM Monomers) werden in 230 ml bei Raumtemperatur gelöst. Es folgt die Zugabe von 0,06% SDS und 0,067g (1,3mol%; 0,44mmol) N,N'-methylen-bis-acrylamid. Die Lösung wird unter Rühren für 30min mit N2 entgast und auf 70°C erwärmt. 0,166g Kaliumpersulfat werden in 20ml gelöst und zur Reaktion gegeben um die Reaktion zu starten. Die Reaktion wird bei 68-70°C für 4h durchgeführt. Nach Abkühlen auf Raumtemperatur wird der Niederschlag für 5 Tage gegen Wasser dialysiert (molecular cut off (MCO) 13.000 Da). Das erhaltene Poly(N-isopropylacrylamid-co-allylamin) zeigt in wässriger Umgebung ein mit steigendem pH-Wert verringertes Quellvermögen.

Ggf. kann in das Hydrogel ein Wirkstoff eingebettet sein.
Matrixpräparation und ggf. Wirkstoffeinbau:
1g des so erhaltenen Polymers werden ggf. mit Verapamil versetzt und mit 0,04g (25wt%) Glutaraldehyd für 2h bei Raumtemperatur vernetzt.

Alternativ kann die Matrixpräparation und ggf. der Wirkstoffeinbettung wie folgt ausgeführt werden: 1g des so erhaltenen Polymers werden ggf. mit Verapamil versetzt. Anschließend werden 0,032g (0,17mmol) 1-Ethyl-3-(3-dimethylaminopropyl)carbodiimid gelöst in 200µl Wasser und 0,015g (0.085mmol) Adipinsäuredihydrazid ebenfalls gelöst in 200µl Wasser bei zugesetzt und für 2h gerührt.

Wirkstoffe, die mit den Vernetzern wie Glutaraldehyd oder EDC reagieren, müssen vor der Reaktion liposomal verkapselt werden.

### Ausführungsbeispiel 2 - Beschichtung eines Stents

Ein Stent aus der biokorrodierbaren Magnesiumlegierung WE43 (4 Gew.% Yttrium, 3 Gew.% Seltenerdmetalle außer Yttrium, Rest Magnesium und herstellungsbedingte Verunreinigungen) wird wie folgt beschichtet:
Der Stent wird von Staub und Rückständen gereinigt und in eine geeignete Stentbeschichtungsapparatur (DES Coater, Eigenentwicklung Fa. Biotronik) eingespannt. Mit Hilfe eines Airbrush Systems (Fa. EFD oder Spraying System) wird der sich drehende Stent unter konstanten Umgebungsbedingungen (Raumtemperatur; 42% Luftfeuchte) halbseitig mit einer der Polymermischungen aus Ausführungsbeispiel 1 oder 2 beschichtet. Bei einem Düsenabstand von 20 mm ist ein 18 mm langer Stent nach ca. 10 min beschichtet. Nach Erreichen der beabsichtigten Schichtmasse wird der Stent 5 min bei Raumtemperatur getrocknet, ehe nach Drehen des Stents und erneutem Einspannen die unbeschichtete Seite auf dieselbe Weise beschichtet wird. Der fertig beschichtete Stent wird für 36 h bei 40°C in einem Vakuumofen (Vakucell; Fa. MMM) getrocknet. Eine Schichtdicke der aufgetragenen Beschichtung beträgt etwa 10 µm.

## Patentansprüche

1. Implantat mit einem Grundkörper, der ganz oder in Teilen aus einem biokorrodierbaren, metallischen Werkstoff besteht, wobei der Werkstoff so beschaffen ist, dass er in wässriger Umgebung zu einem alkalischen Produkt zersetzt wird und wobei der Grundkörper eine Beschichtung aufweist, die aus einem Hydrogel besteht oder dieses enthält, **dadurch gekennzeichnet, dass** das Hydrogel bei erhöhtem pH-Wert ein verringertes Quellvermögen besitzt.

2. Implantat nach Anspruch 1, wobei das Implantat ein Stent ist.

3. Implantat nach einem der vorhergehenden Ansprüche, bei dem der biokorrodierbare, metallische Werkstoff eine Magnesiumlegierung ist.

4. Implantat nach einem der vorhergehenden Ansprüche, wobei das Quellvermögen des Hydrogels bei einem pH-Wert größer als 8 verringert ist.

5. Implantat nach einem der vorhergehenden Ansprüche, wobei das Quellvermögen des Hydrogels bei einem pH-Wert größer als 8 derart vermindert ist, dass das Hydrogel mindestens 30% weniger Wasser aufnehmen kann als bei physiologischem pH-Wert.

6. Implantat nach einem der vorhergehenden Ansprüche, wobei das Hydrogel mindestens eine funktionelle Gruppe aufweist, die mit steigendem pH-Wert einen Übergang von einem ionischen hin zu einem neutralen Ladungszustand zeigt.

7. Implantat nach Anspruch 6, wobei die funktionelle Gruppe ausgewählt ist aus der Gruppe umfassend eine Aminfunktion und eine Amidfunktion.

8. Implantat nach einem der vorhergehenden Ansprüche, wobei das Hydrogel ein Polymer auf Basis von Acrylamid, Methacrylamid, Dimethylaminoethylmethacrylate oder einem Derivat von Acrylamid, Methacrylamid oder Dimethylaminoethylmethacrylate enthält.

9. Implantat nach einem der vorhergehenden Ansprüche, wobei das Implantat eine weitere, äußere Beschichtung aufweist, die ein degradierendes Polymer enthält, bevorzugt aus der Klasse der PLGA (poly(lactic-co-glycolic acid)) oder PLGA-PEG Blockcopolymere.

10. Implantat nach einem der vorhergehenden Ansprüche, wobei die Beschichtung mindestens einen Wirkstoff enthält.

## Claims

1. An implant with a main body consisting completely or partially of a biocorrodible metallic material, wherein the material is such that it decomposes in an aqueous environment to form an alkaline product, and wherein the main body has a coating which consists of or contains a hydrogel, **characterised in that** the hydrogel has a reduced swelling capacity at elevated pH.

2. The implant according to claim 1, wherein the implant is a stent.

3. The implant according to any one of the preceding claims, wherein the biocorrodible metallic material is a magnesium alloy.

4. The implant according to any one of the preceding claims, wherein the swelling capacity of the hydrogel is reduced at a pH greater than 8.

5. The implant according to any one of the preceding claims, wherein the swelling capacity of the hydrogel is reduced at a pH greater than 8, such that the hydrogel can absorb at least 30% less water than at a physiological pH.

6. The implant according to any one of the preceding claims, wherein the hydrogel has at least one functional group which shows a transition between an ionic charge state and a neutral charge state when there is an increase in pH.

7. The implant according to claim 6, wherein the functional group is selected from the group comprising an amine function and an amide function.

8. The implant according to any one of the preceding claims, wherein the hydrogel is selected from a polymer based on acrylamide, methacrylamide, dimethylaminoethyl methacrylate or a derivative of acrylamide, methacrylamide, or dimethylaminoethyl methacrylate.

9. The implant according to any one of the preceding claims, wherein the implant has an additional outer coating containing a degradable polymer, preferably from the class of PLGA (poly(lactic-co-glycolic acid)) or PLGA-PEG block copolymers

10. The implant according to any one of the preceding claims, wherein the coating contains at least one active substance.

## Revendications

1. Implant avec un corps de base qui est constitué totalement ou en partie d'un matériau métallique biocorrodable, où le matériau est tel qu'il est décomposé en un produit alcalin dans un environnement aqueux et où le corps de base présente un revêtement qui est constitué d'un hydrogel ou qui contient celui-ci, **caractérisé en ce que** l'hydrogel possède un pouvoir gonflant réduit pour une valeur de pH élevée.

2. Implant selon la revendication 1, où l'implant est un stent.

3. Implant selon l'une des revendications précédentes, chez lequel le matériau métallique biocorrodable est un alliage de magnésium.

4. Implant selon l'une des revendications précédentes, dans lequel le pouvoir gonflant de l'hydrogel est diminué pour une valeur de pH supérieure à 8.

5. Implant selon l'une des revendications précédentes, dans lequel le pouvoir gonflant de l'hydrogel est diminué pour une valeur de pH supérieure à 8 de telle façon que l'hydrogel peut absorber au moins 30 % d'eau en moins que pour une valeur de pH physiologique.

6. Implant selon l'une des revendications précédentes, dans lequel l'hydrogel arbore au moins un groupe fonctionnel qui présente une transition entre un état de charge ionique et un état de charge neutre avec une valeur de pH en progression.

7. Implant selon la revendication 6, dans lequel le groupe fonctionnel est choisi dans le groupe comprenant une fonction amine et une fonction amide.

8. Implant selon l'une des revendications précédentes, dans lequel l'hydrogel contient un polymère à base d'acrylamide, de méthacrylamide, de diméthylaminoéthylméthacrylate ou d'un dérivé d'acrylamide, de méthacrylamide ou de diméthylaminoéthylméthacrylate.

9. Implant selon l'une des revendications précédentes, où l'implant présente un nouveau revêtement externe qui contient un polymère de dégradation, de préférence, faisant partie de la classe des PLGA (acides poly(lactic-co-glycoliques)) ou des copolymères à blocs PLGA-PEG.

10. Implant selon l'une des revendications précédentes, dans lequel le revêtement contient au moins une matière active.
